# EUROPEAN PATENT APPLICATION

(11) **EP 2 927 831 A1**
(43) Date of publication of application: **07.10.2015**
(21) Application number: 15161903.8
(22) Date of filing: 31.03.2015
(51) Int. Cl.: G06F 19/00

(54) **NETWORK-BASED IDENTIFICATION OF DEVICE USAGE PATTERNS THAT CAN INDICATE THAT THE USER HAS A QUALIFYING DISABILITY**

(30) Priority: 02.04.2014 US 201414243676
(71) Applicant: Avaya Inc., Santa Clara, CA 95054-1233 (US)
(72) Inventor: Michaelis, Paul Roller, Louisville, CO Colorado 80027 (US); Pina, Stacey L., Gahanna, OH Ohio 43230 (US)
(74) Representative: Williams, David John

(57) **Abstract**

The present disclosure is directed to a method, instructions and system to collect accommodation and/or impairment-related information associated with one or more computational devices associated with a user, determine, based on the collected accommodation and/or impairment-related information, that the user potentially requires accommodation of an impairment and/or disability, and perform an action associated with the determination that the user potentially requires accommodation of an impairment and/or disability.

## Description

### FIELD

The disclosure relates generally to network-based systems and methods to identify one or more user characteristics and particularly to network-based systems and methods to identify and provide additional support to a "disabled" user through the communications related accommodations they enable.

### BACKGROUND

Globally, employers are under increasing regulatory pressure to increase the employment and retention of "disabled" citizens or citizens with a disability. For many years, US state and federal laws, such as the Americans with Disabilities Act and Section 503 of the Rehabilitation Act, made very specific requirements for employers to provide reasonable accommodations to qualified disabled employees or employees with a disability and to comply with Affirmative Action requirements to track good-faith efforts for the hiring and retention of individuals who self-identify as disabled or having a disability.

Today, US and global labor statistics and census data establish some sobering facts about the increase in disabilities among children and adults. In the US, 20% of adults are estimated to have some form of disability, compared to more than 15% worldwide. The reported unemployment rate for Americans with a disability is double the unemployment rate of Americans not having a disability. Looking to the future, the US Social Security Administration estimates that 1 in 4 of today's 20 year olds will have a disability by the time they retire. Many of those disabilities will be related to communication, chiefly vision and hearing impairments.

To target the disproportionate percentage of Americans with a disability in the unemployment ranks, the US Department of Labor recently updated Section 503 of the Rehabilitation Act Amendments. Changes include a mainstreaming of the definition of "disability" and increased requirements for all US federal contractors and sub-contractors. These requirements include: 1) tracking applicants and employees having a disability in a more rigorous manner, 2) requiring all contractors to establish a benchmark of 7% employees having a disability in all job groups within their US employee base and 3) requiring contractors to provide more detailed reports on the accommodations offered to employees having a disability. A failure to comply can jeopardize the continuance of the contract.

A complication for impacted organizations is the fact that privacy laws, such as HIPAA, protect the confidentiality of medical information. Employers often cannot discover what proportion of their employees have a qualifying disability because they are limited to the information that employees provide voluntarily. In most cases, employees do not have an incentive to self-identify unless they need to request a significant accommodation. In some cases, there is a reluctance to self-identify that is caused by employees' belief that the self-identification would harm their job security or career prospects. (In reality, due to Federal anti-discrimination regulations, self-identification as having a qualifying disability may be more likely to protect job security rather than harm it.)

There is a need to determine, track, and/or report the proportion of employees having a qualifying disability.

### SUMMARY

These and other needs are addressed by the various aspects, embodiments, and/or configurations of the present disclosure. The present disclosure is directed to identification of a user having an accommodation and/or impairment and/or disability based on devices associated with the user.

A method, instructions, and system, according to this disclosure, can perform the following steps, tasks, and operations:
(a) Collect accommodation and/or impairment-related information associated with one or more computational devices (such as a (tele)communication device, assistive technology, and the like) associated with a user;
(b) determine, based on the collected accommodation and/or impairment-related information, that the user potentially has an impairment and/or disability; and
(c) perform an action associated with the determination that the user potentially has an impairment and/or disability.

The accommodation and/or impairment-related information can include one or more of an enabled feature, a disabled feature, a setting, and an assistive technology. By way of example, the accommodation and/or impairment-related information can relate to use by the user of one or more of sticky keys, toggle keys, filter keys, mouse keys, sound or volume settings, font selection, formatting, font, text, and/or icon size, screen magnification, visual indication of sounds, changed computer sounds, text and/or background colors, contrast, color and transparency of window borders, thickness of focus rectangle, disablement of unnecessary animations, read mode, speak, remove background images, narration, audio description, on-screen keyboard, color, size, and/or thickness of the on-screen mouse pointer, scroll speed of the mouse wheel, keyboard settings, keyboard shortcuts, time period for how long notification dialog boxes remain open, Internet accessibility, a screen magnifier, a screen reader, a Braille printer, a Braille note taker, a Braille embosser, a book reader, an add-in to convert documents to DAISY format for a book reader, a speech synthesizer, a speech recognition program, a touch keyboard, an on-screen keyboard, a mouse, a joystick, a trackball, a keyboard filter, an alternative PC hardware or all-access workstations, an alternative input device, a sign language interpretation device, a personal listening device, a personal amplification device, a sign language translator, a word prediction program, a reading tool, a learning disability program, an augmentative and assistive communication device, a bone-conduction phone, an amplified phone, and a text telephone.

The method, instructions, or system can identify patterns of device usage (including usage of the features, settings, and/or devices noted above) by users that might be indicative of an impairment and/or disability.

The action can include one or more of provide an instructional message to the selected user, recommend a feature, setting, and/or configuration of a computational device associated with the user, track the accommodation and/or impairment and/or disability for future reporting to a governmental entity, provide the user with a human or automated agent specially skilled to interact with the user, encourage the user to indicate in a job application that he or she has accommodation and/or impairment and/or disability, and provide the user with targeted advertisements regarding products or services for users having the impairment and/or disability.

The determining step, task, or operation can include one or more of determining whether the collected accommodation and/or impairment-related information is attributable to the user or a physical environment of the user and determining whether the collected accommodation and/or impairment-related information has occurred for at least a predetermined period of time.

The determining step, task, or operation can include one or more of determining a type of the accommodation and/or impairment and/or disability, determining a severity of the impairment and/or disability, and determining a likelihood that the user has the impairment and/or disability.

The method, instructions or system can perform additional steps, tasks, or operations including:
mapping enabled and disabled features and/or settings of a first communication device associated with the user to the features and/or settings of a second communication device associated with the user; and
changing at least some of the enabled and disabled features and/or settings of the second communication device to corresponding ones of enabled and disabled features and/or settings of the first communication device.

The disclosure can provide a centralized resource capable of identifying employee usage patterns that are suggestive of a qualifying disability. Using equipment of Avaya Inc.™ to illustrate the solution, an Aura™ system could detect the accessibility adjuncts (e.g., TTY devices and/or Universal Access Phone Status™ software of Avaya Inc™) and the accessibility settings (e.g., large font display and/or consistently high amplitude settings) of Aura™-connected devices, thereby allowing employees with a potentially qualifying disability to be identified. Avaya Universal Access Phone Status™ software is an accessibility adjunct, intended primarily for people who are blind or visually impaired. The software is commonly loaded onto the user's desktop personal computer or laptop. The status of the user's telephone is then monitored via a connection to an Avaya Media Server running Avaya Aura® Communication Manager.

Based on the expanded definition of "disability" under the Americans with Disabilities Act Amendments, two examples using Model 9641™ telephones of Avaya Inc.™ illustrate potentially qualifying disabilities. In the first example, an employee who enables the "large font" mode on the phone's display may have a qualifying visual disability. In the second example, an employee who consistently has the handset volume set to a higher-than-typical level, or who has swapped out the standard handset for an Avaya S1-K5™ amplified handset, may have a qualifying hearing disability. While these examples are for telephony products, the present disclosure is not restricted to telecommunication systems. There are, for instance, many user-selectable accessibility options on personal computers and in browsers, the enablement of which could be detected and acted upon by a network-based resource. Illustratively, a PC display option often used by people with certain types of visual impairment (such as cataracts) is "high contrast, reverse video" mode, in which a document or web page is presented as white or green text against a black background instead of as black text against a white background. In addition to identifying users who may have a qualifying disability, a capability of this sort could also facilitate what is, in essence, assistive notifications: "It has been detected that you have enabled high contrast reverse video on your PC but not on your Avaya telephone. Did you know that there is a high contrast reverse video option on your Avaya telephone?"

The present disclosure can provide a number of advantages depending on the particular aspect, embodiment, and/or configuration. The system and method of the present disclosure can detect patterns of usage of devices that might be indicative of accommodated communication and/or an underlying qualifying disability, thereby making it easier for employers to offer and track accommodations and demonstrate compliance with government-mandated disability-related employment objectives (e.g., enable government contractors and subcontractors to demonstrate movement toward the mandated 7% employment benchmark). The system and method can identify employees with qualifying disabilities and thereby ensure that the employee will receive the job protections guaranteed by law while also preserving the employer's federal contracts and therefore employment for other employees. By identifying employees with qualifying disabilities, the system and method can enable the employer more effectively to offer accommodations that would improve employees' job performance.

These and other advantages will be apparent from the disclosure.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

"Accessible technology" refers to any computer technology that users can adjust and/or employ to accommodate his or her vision, dexterity, hearing, cognitive, language, learning, and/or speech needs. Accessibility technology can be in the form of accessibility features or settings built into software programs and specialty hardware devices or software programs.

"Assistive technology" refers to any technology that users can adjust and/or employ to accommodate his or her vision, dexterity, hearing, cognitive, language, learning, and/or speech needs. Examples of assistive technology include accessible technology, adjuncts, peripherals, plug-ins, and add-ins.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material".

The term "computer-readable medium" as used herein refers to any storage and/or transmission medium that participate in providing instructions to a processor for execution. Such a medium is commonly tangible and non-transient and can take many forms, including but not limited to, non-volatile media, volatile media, and transmission media and includes without limitation random access memory ("RAM"), read only memory ("ROM"), and the like. Non-volatile media includes, for example, NVRAM, or magnetic or optical disks. Volatile media includes dynamic memory, such as main memory. Common forms of computer-readable media include, for example, a floppy disk (including without limitation a Bernoulli cartridge, ZIP drive, and JAZ drive), a flexible disk, hard disk, magnetic tape or cassettes, or any other magnetic medium, magneto-optical medium, a digital video disk (such as CD-ROM), any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored. Computer-readable storage medium commonly excludes transient storage media, particularly electrical, magnetic, electromagnetic, optical, magneto-optical signals.

The term "database" refers to an organized collection of data. The data are typically organized to model relevant aspects of reality in a way that supports processes requiring this information.

The term "database management system" or DBMS refers to a specially designed application that interacts with the user, other applications, and the database itself to capture and analyze data. A general-purpose database management system (DBMS) is a software system designed to allow the definition, creation, querying, update, and administration of databases. Well-known DBMSs include MySQL™, MariaDB™, PostgreSQL™, SQLite™, Microsoft SQL Server™, Oracle, SAP™, dBASE™, FoxPro™, IBM DB2™, LibreOffice Base™ and FileMaker Pro™.

The terms "determine", "calculate" and "compute," and variations thereof, as used herein, are used interchangeably and include any type of methodology, process, mathematical operation or technique.

The term "disability" refers to the consequence of an impairment that may be physical, cognitive, intellectual, mental, sensory, emotional, developmental, or some combination of these. By way of illustration, Section 503 defines "disability" as an impairment that substantially limits a major life activity, even if it were not to limit any other major life activity, or an impairment that is episodically active or in remission and would substantially limit a major life activity when active.

The term "means" as used herein shall be given its broadest possible interpretation in accordance with 35 U.S.C., Section 112, Paragraph 6. Accordingly, a claim incorporating the term "means" shall cover all structures, materials, or acts set forth herein, and all of the equivalents thereof. Further, the structures, materials or acts and the equivalents thereof shall include all those described in the summary, brief description of the drawings, detailed description, abstract, and claims themselves.

The term "module" as used herein refers to any known or later developed hardware, software, firmware, artificial intelligence, fuzzy logic, or combination of hardware and software that is capable of performing the functionality associated with that element.

A "server" is a system (software and suitable computer hardware) that responds to requests across a computer network to provide, or help to provide, a network service.

A "switch" is a device that channels incoming data from any of multiple input ports to the specific output port that will take the data toward its intended destination.

A "web browser" is a software application for retrieving, presenting and traversing information resources on the Internet. An *information resource* is identified by a Uniform Resource Identifier (URI/URL) and may be a web page, image, video or other piece of content. Hyperlinks present in resources enable users easily to navigate their browsers to related resources.

A "web server" is the hardware (the computer) and/or the software (the computer application) that helps to deliver web content that can be accessed through the Internet.

The preceding is a simplified summary of the disclosure to provide an understanding of some aspects of the disclosure. This summary is neither an extensive nor exhaustive overview of the disclosure and its various aspects, embodiments, and/or configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and/or configurations of the disclosure are possible utilizing, alone or in combination, one or more of the features set forth above or described in detail below. Also, while the disclosure is presented in terms of exemplary embodiments, it should be appreciated that individual aspects of the disclosure can be separately claimed.

### BRIEF DESCRIPTION OF THE DRAWINGS

Fig. 1 is a block diagram of an architecture according an embodiment of the disclosure;
Fig. 2 is a flow schematic according to an embodiment of the disclosure;
Fig. 3 is a flow schematic according to an embodiment of the disclosure;
Fig. 4 is a flow schematic according to an embodiment of the disclosure; and
Fig. 5 is a flow schematic according to an embodiment of the disclosure.

### DETAILED DESCRIPTION

### The Network-Based Disability Reporting System

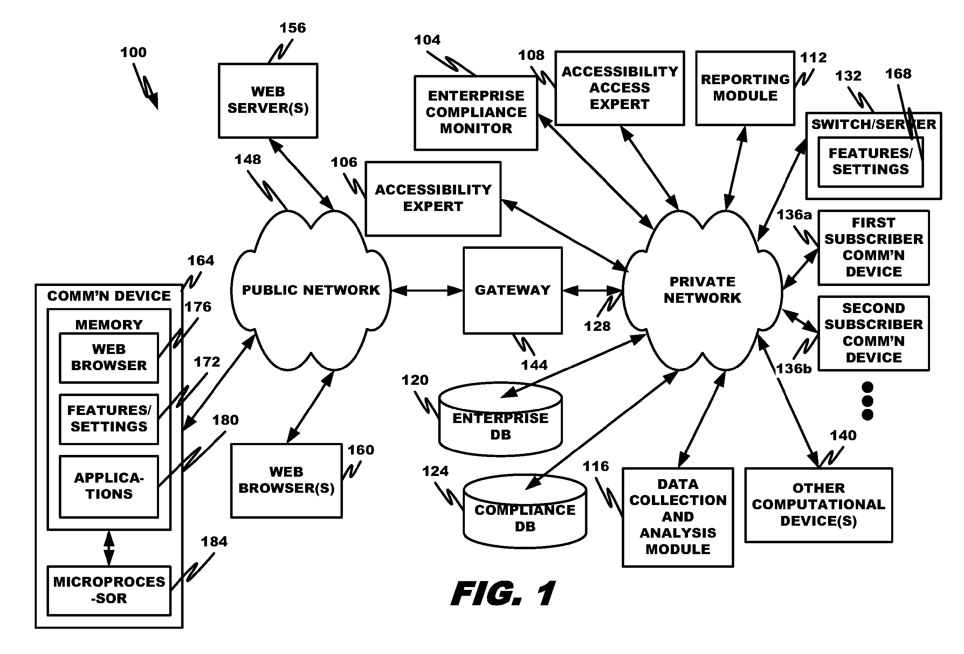

Fig. 1 depicts a network-based disability reporting system 100 according to an embodiment of the disclosure. The system 100 includes an enterprise compliance monitor 104, accessibility expert 106, accessibility access expert 108, reporting module 112, data collection and analysis module 116, enterprise database 120, and compliance database 124 in communication, via private or trusted network 128, with switch/server 132, first, second, ... subscriber communication devices 136a, b, ..., other computational devices 140, and, via gateway 144 and public or untrusted network 148, , web server(s) 156, web browser(s) 160, and external communication device(s) 164.

The switch/server 132 is conventional and controls the first, second, ... subscriber communication devices 136a, b, ... Examples include the S8300™, S8500™, and S8710™ media servers and Communication Server 2100™, of Avaya Inc. In some applications, the switch/server 132 includes features and settings 168 for the first, second, ... subscriber communication devices 136a, b, ...

The first, second, ... subscriber communication devices 136a, b, ... can be any communication devices, including telephones, personal computers, laptops, tablet computers, cellular phones, personal digital assistants, and the like. The subscriber communication devices are controlled by the switch/server 132 and typically correspond to a person associated with an enterprise maintaining the private network 128, or enterprise network.

The other computational devices 140 include other types of networked devices including text, voice, and multimedia messaging servers, facsimile machines, telephony adjuncts such as TTY or text telephones, and non-subscriber communication devices wirelessly accessing the private network 128.

The private or trusted network 128 is a network that commonly uses private IP address space.

The gateway 144 is a router or a proxy server that routes between the private and public networks 128 and 148. Examples include G700™, G350™, and G430™ of Avaya Inc.

The external communication device 164 can be any communication device, including personal computers, laptops, tablet computers, cellular phones, personal digital assistants, and the like. A typical communication device 164 includes a web browser 176, features and settings 172 (e.g., feature enablement or disablement and/or feature values), and applications 180 executed by a microprocessor 184.

The web browser(s) 160 and web server(s) 156 are conventional.

The public network 148 is an untrusted network, such as the Internet.

### The Data Collection and Analysis Module 116

The data collection and analysis module 116 collects accommodation and/or impairment-related information, such as features and settings 168 and features and settings 172, from a variety of sources associated with a selected user, such sources including the switch/server 132, first, second, ... subscriber communication devices 136a,b ..., other computational device(s) 140, (external) communication device 164, and web browser(s) 160, and analyzes the collected information to identify users having a potential disability and the type of disability. Accommodation and/or impairment-related information can include not only activated and/or non-activated communication or computational device features, settings, capabilities, and configurations and adjuncts, peripherals, plug-ins, add ins, and other assistive technologies but also observed user behavior patterns.

Examples of activated and/or non-activated communication or computational device features, settings, capabilities, and configurations indicative of an accommodation and/or impairment and disability include sticky keys (which allow the user to enter a combination of multiple keys without having to hold a first key down when he or she depresses a second key), toggle keys (which play an alert each time the user presses Caps Lock, Num Lock, or Scroll Lock keys), filter keys (which causes the computer to ignore keystrokes that occur in rapid succession or keystrokes held down for several seconds unintentionally), mouse keys, sound or volume settings, font selection, formatting, font, text, and/or icon size, screen magnification, visual indication of sounds (e.g., using text and/or visual cues instead or in lieu of sounds, such as Sound Sentry™), changed computer sounds, text and/or background colors, contrast (e.g., high contrast making displayed objects easier to see), color and transparency of window borders (making the borders easier to see), thickness of focus rectangle (around the currently selected item in a dialog box), disablement of unnecessary animations, read mode (which hides most of the buttons and tools from the display), speak (which enables user to speak a command rather than inputting the command through tactile input), remove background images, narration (or automated text-to-speech translation or conversion of displayed text), audio description (which can describe what is happening in a video), on-screen keyboard (which enables typing using a mouse or other pointing device, such as a joystick, by selecting keys from a picture of a keyboard), color, size, and/or thickness of the on-screen mouse pointer, scroll speed of the mouse wheel, keyboard settings (e.g., select how long you need to press a key before the keyboard character starts repeating, the speed at which keyboard characters repeat, and the rate at which the cursor blinks, etc.), keyboard shortcuts (which are combinations of two or more keys that, when pressed, can be used to perform a task that would typically require a mouse or other pointing device), time period for how long notification dialog boxes remain open, Internet accessibility (which ignores colors, font styles and font sizes used on web pages, or formats web pages using a user specified style sheet), and other assistive features, settings, capabilities, and configurations.

Examples of assistive adjuncts, peripherals, plug-ins, add ins, and other active and/or disabled hardware and software devices and modules indicative of a disability include screen magnifiers, screen readers (e.g., a software program that presents graphics and/or text as speech such as Mini Translator™ which enables a user to select a word or phrase and receive an audio pronunciation of the word or phrase), Braille printers, Braille note takers, Braille embossers, book readers, add-in to convert documents to DAISY format for a book reader, speech synthesizers (or text-to-speech application), speech recognition programs (e.g., speech-to-text recognition or conversion applications), touch keyboards, on-screen keyboards, mice, joysticks, trackballs, keyboard filters (e.g., typing aids, such as word prediction utilities and add-on spelling checkers that can be used to reduce the number of required keystrokes), alternative PC hardware or all-access workstations, alternative input devices (e.g., alternative keyboards, electronic pointing devices, sip-and-puff systems, and wands and sticks that allow individuals to control their computers through means other than a standard keyboard or pointing device), sign language interpretation devices, personal listening devices, personal amplification devices, sign language translators (such as iCommunicator™ which is a graphical sign language translator that converts speech to sign language in real time, thereby enabling people who are deaf to communicate more easily with hearing people), word prediction programs (which allow the user to select a desired word from an on-screen list located in the prediction window), reading tools and learning disability programs (which make text-based materials more accessible for people who struggle with reading), augmentative and assistive communication devices (which enables the user to type in a word, phrase, or sentence to communicate-or select a series of symbols or pictures on the device-and the device "speaks" aloud for the user), bone-conduction phone, amplified phone, text telephone (e.g., Teletype ("TTY") or Telecommunications Device for the Deaf ("TDD") phone), Universal Access Phone Status™ software of Avaya Inc., and other types of accessible technology.

Examples of observed user behavior patterns that may be indicative of a disability include using a keyboard rather than a mouse to navigate and select items on a web page (possibly indicating that the user cannot work a mouse, as would be the case if the user were blind), zooming in or enlarging displayed content (such as zooming in on or enlarging a webpage), activating a window by hovering over it (rather than clicking the mouse), using fingers to scroll, resize windows, play media, and pan and zoom, and frequency and/or duration of usage of any of the above disability assistive software and devices.

The collected or sensed accommodation and/or impairment-related information can be compared by the module 116 against templates, each template corresponding to differing types and/or degrees of severity of disability, to determine whether or not the user potentially has a disability, what type of disability the user potentially has, and to what degree the user potentially is impaired by the disability. The templates map the types of accommodation and/or impairment-related information associated with differing types of disabilities and optionally the degree of severity of the corresponding type of disability.

Based on the collected accommodation and/or impairment-related information and rules and templates, the data collection and analysis module 116 can determine a type of a potential disability of the user. By way of example, users with vision impairments, such as low vision, blindness, or color blindness) can be identified by use of one or more of screen magnification, high contrast (e.g., between text and background colors), large font size and/or icon size (e.g., without changing screen resolution), a screen reader (or other text-to-speech program), speech recognition software (such as to operate the computer and/or software), enablement of a read mode, keyboard web page navigation, a Braille printer, a Braille display, a Braille embosser, a Braille note taker, a book reader, an add-in to convert documents to DAISY format, and the like. Hearing impairments, such as hearing loss, hard-of-hearing, and deafness, can be identified by use of one or more of text or visual alternatives for sounds, high volume levels, changed computer sounds, sign language interpretation or translation, a personal listening device, a text phone, and the like. Dexterity and/or mobility impairments, such as caused by arthritis, cerebral palsy, multiple sclerosis, loss of limb or digit, spinal cord injury, and repetitive stress injury, can be identified by use of one or more of mouse settings (e.g., regarding mouse button configuration and timing (such as double-click speed), to make the mouse pointer more visible, and to alter the scroll speed of the mouse wheel and/or how quickly the mouse pointer responds to movements of the mouse), an increased size of a mouse-selectable screen element to provide a larger target, mouse keys to move the mouse pointer, sticky keys, toggle keys, filter keys, keyboard shortcuts, access keys, keyboard settings, an on-screen keyboard, speech recognition software (such as to dictate into almost any application (e.g., the user can dictate documents and email and surf the web by voice command)), a touch-screen monitor (such as to scroll, resize windows, play media, and pan and zoom), disablement of automatic arrangement of windows when the mouse cursor is moved to the edge of the screen, enablement of activate a window by hovering over it with the mouse cursor, a touch screen, keyboard web page navigation, a disability assistive keyboard (such as an alternative keyboard) and/or a mouse, joystick, trackball, keyboard filter, alternative PC hardware, all-access workstation, alternative input device, and the like. Language and/or communication impairments, such as aphasia, delayed speech, dyslexia, and other conditions resulting in difficulties remembering, solving problems, and/or perceiving sensory information, can be identified by use of one or more of sticky keys, toggle keys, filter keys, enablement of remove background images, enablement of disablement of all unnecessary animations, long period for notification dialog boxes to stay open, a touch screen, a keyboard filter, speech recognition software, enablement of a read mode, a screen reader, keyboard web page navigation, an augmentative and/or assistive communication device, a speech synthesizer, and the like. Learning impairments can be identified by use of a word prediction program, a reading tool, a learning disability program, a speech synthesizer, and a speech recognition program.

The data collection and analysis module 116 can further determine a likelihood or probability that the user has a selected type of impairment. This can be done by determining whether multiple accommodation and/or impairment indicators point to a selected impairment and the duration or persistency of the accommodation and/or impairment indicator for the user. As shown above, one accommodation and/or impairment indicator can point to multiple types of impairment. The module 116 can avoid false positives by determining that multiple accommodation and/or impairment indicators apply to the selected user and which accommodation and/or impairment indicators for the multiple possible impairments do not apply to the selected user. Illustratively, a person who is detected to be using a text-to-speech screen-reading adjunct might be blind or might be dyslexic. A person who is detected to be using a keyboard, and never a mouse, might be blind or might have a motor control dysfunction. It might be reasonable to infer that a person using a text-to-speech screen-reading adjunct, who always uses a keyboard and never a mouse, is blind.

The data collection and analysis module 116 can further determine a degree of severity of the impairment based on those accommodation and/or impairment indicators applying to the selected user and those which do not apply to the selected user. For example, a first set of accommodation and/or impairment indicators may indicate at least a first degree of impairment but a second set of accommodation and/or impairment indicators would indicate a higher second degree of impairment. If only one or more accommodation and/or impairment indicators from the first set apply to a selected user and no accommodation and/or impairment indicator from the second set applies, the module 116 can assume that the selected user has the first but not the second degree of impairment.

The data collection and analysis module 116 can further determine, based on the impairment, whether or not the user has a disability as defined by governing rules, policies, and/or laws. For example, the data collection and analysis module 116 can determine whether or not the user has a qualifying disability.

The data collection and analysis module 116, after determining which of the accommodation and/or impairment indicators apply to a selected user and any other accommodation and/or impairment-related information noted above, stores the accommodation and/or impairment-related information in one or both of the enterprise and/or compliance databases 120 and 124 with a suitable timestamp.

### The Accessibility Expert 106

The accessibility expert 106 provides advice, recommendations, or instructions to a user determined by the data collection and analysis module 116 to have a potential disability. The advice, recommendations, or instructions pertain to the disability-related legal rights of and benefits to the user and/or requirements of and benefits to the enterprise, such as regarding reasonable accommodations required to be offered to employees having a qualified disability (such as Section 255 of the Telecommunication Act of 1996 and the Communications and Video Accessibility Act Amendments of 1998), Affirmative Action requirements (such as under Section 508 of the Rehabilitation Act Amendments of 1998), government contractor requirements, other legally guaranteed employee job benefits and protections, and other legal or enterprise mandated rights and benefits. By providing the user with advice, recommendations, or instructions, the accessibility expert 106 seeks to encourage the user to self-identify his or her disability to the enterprise.

The accessibility expert 106 could provide, for example, an automated, confidential message to the user with customizable text such as:
It has been detected that you tend to have your handset amplitude set to a very high level. If you would like information on additional accommodations and
enhancements that may assist you in performing the essential functions of your job, please contact <phone number> or <email>. Please note also that your company is an equal opportunity employer and a government contractor, legally obligated to report the proportion of employees who have a qualifying disability. For information on how to confidentially self-identify, to assist with your employer's mandatory government reporting of employment statistics, please visit http://<URL>.

As will be appreciated, other automated confidential messages can be provided to the user depending on the needs of the enterprise.

### The Enterprise Compliance Monitor 104

The enterprise compliance monitor 104 determines, based on identified actual or potential disabilities, a degree of compliance of the enterprise with enterprise disability-related policies and/or legal requirements. It can access both confidential employment records and the confidential data and analysis of the module 116 maintained in one or more of the enterprise and compliance databases 120 and 124. The confidential nature of the data and other records can be maintained in accordance with legal requirements by restricted employee access and other security measures. For example, to protect the privacy of employees, the enterprise compliance monitor 104 can provide the names of employees identified as having potential disabilities only to human resources. The enterprise compliance monitor 104 can prepare differing reports regarding compliance. For example, the enterprise could demonstrate a good-faith compliance effort to the governmental entity simply by reporting: "Approximately 3% of our employees in job group XX have been detected to have telephone usage patterns that are consistent with a qualifying level of hearing loss." The report content can be adapted to comply with any pertinent enterprise policies and/or governmental requirements. The enterprise compliance monitor 104 and data collection and analysis module 116 collectively provide a centralized resource that can allow employees with a potentially qualifying disability to be identified automatically based, for example, on the features and settings and adjuncts they have selected.

### The Accessibility Access Expert 108

The accessibility access expert 108 can provide assistance to the user to configure his or her communication and other devices to accommodate better access for his or her impairment and/or disability and/or automatically adjust features and settings and otherwise reconfigure a communication or other device to reflect default or customized features and settings of a different device.

The type of assistance provided depends on the particular impairment and/or disability involved. For example, if the accessibility access expert 108 were to determine from the data collection and analysis module 116 that the user has a particular impairment and/or disability, the accessibility access expert 108 can recommend other feature or device settings and/or accessible technology that may provide improved access for the user. By way of illustration, instructions to users with vision impairments can be one or more of the use of screen magnification, high contrast (e.g., between text and background colors), large font size and/or icon size (e.g., without changing screen resolution), a screen reader (or other text-to-speech program), speech recognition software (such as to operate the computer and/or software), enablement of a read mode, keyboard web page navigation, a Braille printer, a Braille display, a Braille embosser, a Braille note taker, a book reader, add-in to convert documents to DAISY format, and the like. Instructions to users with a hearing impairment can be one or more of the use of text or visual alternatives for sounds, high volume levels, changed computer sounds, sign language interpretation or translation, a personal listening device, a text phone, and the like. Instructions to users with a dexterity and/or mobility impairment can be one or more of the use of particular mouse settings (e.g., mouse button configuration and timing (such as double-click speed), to make the mouse pointer more visible, and to alter the scroll speed of the mouse wheel or how quickly the mouse pointer responds to movements of the mouse), an increased size of a mouse-selectable screen element to provide a larger target, mouse keys to move the mouse pointer, sticky keys, toggle keys, filter keys, keyboard shortcuts, access keys, keyboard settings, an on-screen keyboard, speech recognition software (such as to dictate into almost any application (e.g., the user can dictate documents and email and surf the web by voice command)), a touch-screen monitor (such as to scroll, resize windows, play media, and pan and zoom), disablement of the automatic arrangement of windows when the mouse cursor is moved to the edge of the screen, enablement of activate a window by hovering over it with the mouse cursor, keyboard web page navigation, a disability assistive keyboard (such as an alternative keyboard) and/or mouse, a joystick, a trackball, a keyboard filter, alternative PC hardware, an all-access workstation, an alternative input device, and the like. Instructions to users with a language and/or communication impairment can be one or more of the use of sticky keys, toggle keys, filter keys, enablement of remove background images, disablement of all unnecessary animations, long period for notification dialog boxes to stay open, a touch screen, a keyboard filter, speech recognition software, enablement of a read mode, a screen reader, keyboard web page navigation, an augmentative and/or assistive communication device, and a speech synthesizer, and the like. Instructions to users with a learning impairment can be one or more of the use of a word prediction program, a reading tool, a learning disability program, a speech synthesizer, and speech recognition programs.

The accessibility access expert 108 can, using inter-device mappings of features and settings, automatically enable/disable features and adjust settings and otherwise reconfigure a communication or other device to reflect default or customized set of enabled/disabled features and settings of a different device. An example of such a feature/setting mapping of first feature and/or setting values in a first device to a similar first feature and/or setting value in a different device, could involve one or more of sticky keys, toggle keys, filter keys, mouse keys, sound or volume settings, font selection, formatting, font, text, and/or icon size, screen magnification, visual indication of sounds, computer sounds, text and/or background colors, contrast, color and transparency of window borders, thickness of focus rectangle, turn off unnecessary animations, read mode, speak, remove background images, narration, audio description, on-screen keyboard, color, size, and/or thickness of the on-screen mouse pointer, scroll speed of the mouse wheel, keyboard settings, keyboard shortcuts, time period for how long notification dialog boxes remain open, and Internet accessibility.

### The Reporting Module 112

The reporting module 112, using input from the enterprise compliance monitor 104, can provide, via the network transmission, accommodation and/or impairment-related information to a secure server 156 for tracking and reporting purposes. The accommodation and/or impairment-related information can be in a form and content required by the responsible government entity. For example, the accommodation and/or impairment related information can track accommodation, qualifying disabilities and potential qualifying disabilities as a percentage of total employment for a specific job category.

The various accommodation and/or impairment and/or disability-related modules, using publicly accessible web browser features and settings 160 on a user's communication device, report an accommodation and/or potential impairment and/or disability to an appropriate non-governmental entity, such as a business. For example, a job applicant accessing a potential employer's web site can be identified, by the data collection and analysis module 116, as having a potential impairment and/or disability based on his or her browser features and settings. The enterprise compliance monitor 104 could provide instructions to the user on self-identifying his or her impairment and/or disability in an employment application to receive potentially preferential employment consideration. In a further example, a potential customer accessing a business web site can be identified, by the data collection and analysis module 116, as having a potential impairment and/or disability based on his or her browser features and settings. Advertisements directed to products or services for the identified impairment and/or disability could be provided to the user, via user accessed web pages, to encourage the user to purchase the products or services.

### Enterprise Disability Monitoring and Reporting

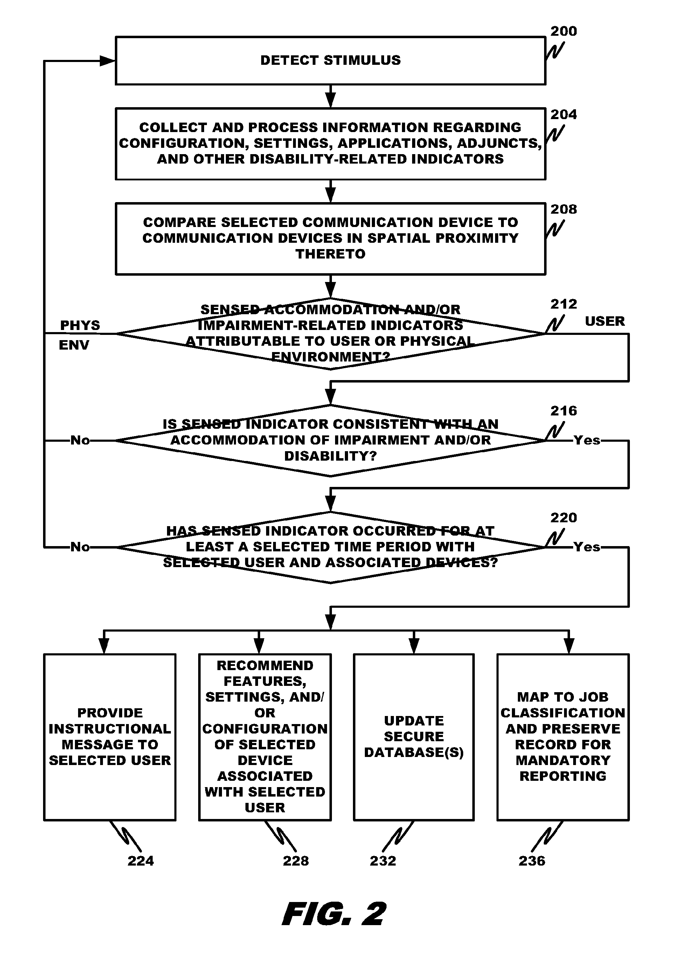

With reference to Fig. 2, the operation of the various modules will be discussed with reference to enterprise compliance with disability policies, rules, and requirements.

In step 200, the data collection and analysis module 116 detects a stimulus, such as a time-based interrupt, a user accessing the private network 128 via or operating a first, second, ... subscriber communication device 136a, b, ..., other computational device 140, and the like, provisioning of a new first, second, ... subscriber communication device 136a, b, ... or other computational device 140, or another suitable stimulus.

In response, the data collection and analysis module 116, in step 204, collects and processes information regarding configuration, settings, enabled or disabled features, applications, adjuncts and other accessibility technology, and other accommodation and/or impairment indicators. This can be done by querying the switch/server 132 with respect to subscriber devices, the devices themselves, the enterprise database for personnel records (which may contain accommodation and/or impairment-related information), the compliance database for relevant data from prior data collection, processing, and analysis by the module 116, and the like. The data is processed as noted above to identify users having potential impairments and/or disabilities, the type and severity of impairments and/or disabilities, and a likelihood or probability that the foregoing information is accurate and reliable.

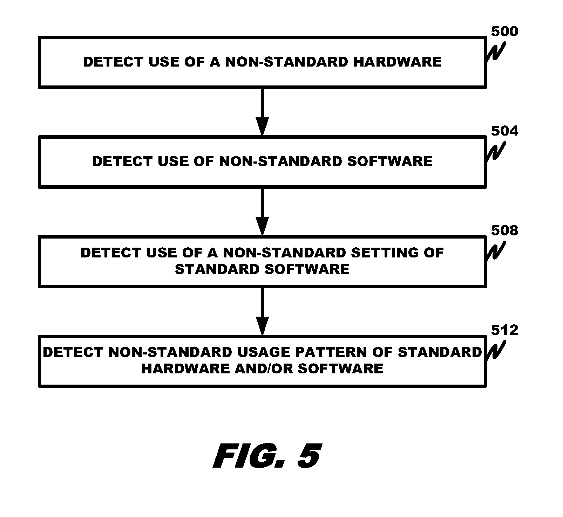

Fig. 5 depicts one approach to collecting accommodation and/or impairment information and indicators. In step 500, the data collection and analysis module 116 attempts to detect that a non-standard device is being used by the user (e.g., an amplified handset, TTY device, or other type of hardware accessibility technology). In step 504, the data collection and analysis module 116 attempts to detect that non-standard software is being used by the user (e.g., the JAWS text-to-speech screen reader, Avaya "Universal Access Phone Status" software, or other type of assistive technology). In step 508, the data collection and analysis module 116 attempts to detect that a non-standard setting or feature of standard software is being used (e.g., reverse video, telephone amplitude consistently set to a high level, and the like). Finally, in step 512 the data collection and analysis module 116 attempts to detect a non-standard usage pattern of standard hardware and software. For example, keeping in mind that it is much easier to navigate a web page with a mouse rather than via the keyboard, detection that someone is using a keyboard for web page navigation could be an indicator that the user is unable to work a mouse. Other patterns that could indicate the presence of a disability include someone taking much longer than an expected amount of time to complete certain tasks (e.g., typing very slowly into a text entry field), frequently asking for IVR options to be repeated, depressing telephone keys for an unusual amount of time (as measured by DTMF tone duration), and frequently undoing or erasing previously entered responses.

Referring again to Fig. 2 in step 208, the data collection and analysis module 116 compares a selected communication device having settings or enabled or disabled features indicating a potential user impairment and/or disability with one or more communication devices associated with a different user in spatial proximity to the selected communication device to determine whether the settings or enabled or disabled features are related to an environmental factor as opposed to a user impairment and/or disability. For example, the settings or enabled or disabled features can be due to environmental disruptions or variations, such as ambient noise levels, lighting problems, sunlight, and the like. The spatial proximity of the various communication devices can be based on network topology stored in the enterprise database.

In decision diamond 212, the data collection and analysis module 116 determines whether a sensed accommodation and/or impairment indicator is attributable to a user impairment or physical environment. When multiple spatially proximal devices, including the selected communication device, associated with different users have similar settings and/or enabled or disabled features indicating a type of accommodation and/or impairment, the settings and/or enabled or disabled features can be assumed to result from the surrounding physical environment and not from a user disability. In this manner, the settings and/or enabled or disabled features are disregarded in the data process and analysis and/or used to reduce a likelihood or probability that the user has a disability associated with the impairment.

When the sensed accommodation and/or impairment indicator is determined to be attributable to the user and not to the physical environment, the data collection and analysis module 116 proceeds to decision diamond 216 and determines whether the sensed accommodation and/or impairment indicator is consistent with accommodation of a disability. This determination is commonly made using rules and/or templates associated with specific disabilities or impairments. For example, a first set of rules associates a sensed accommodation and/or impairment indicator with a first type of disability or impairment. Likewise, a first template maps a sensed accommodation and/or impairment indicator to an associated first type of disability or impairment. Examples of rules and templates are provided above in connection with features, settings, and assistive technologies associated with different types of impairments and/or disabilities.

When the sensed accommodation and/or impairment indicator is consistent with accommodation of a potential disability, the data collection and analysis module 116 proceeds to decision diamond 220 where the data collection and analysis module 116 determines whether the sensed accommodation and/or impairment indicator is persistent and/or consistent with the selected user and/or device associated with the selected user. This can be done based on previously collected and time stamped data. Where consistency and/or persistency is determined to exist due to the sensed accommodation and/or impairment indicator having been in use by the selected user for a specified time period, there is a greater likelihood that the selected user has a disability associated with the sensed accommodation and/or impairment indicator.

When the sensed accommodation and/or impairment indicator is determined to be associated with the physical environment (decision diamond 212), the sensed accommodation and/or impairment indicator is determined not to be consistent with accommodation of a qualifying disability (decision diamond 216), or the sensed accommodation and/or impairment indicator is not determined to be persistent and/or consistent (decision diamond 220), the data collection and analysis module 116 returns to step 200.

When the sensed accommodation and/or impairment indicator is determined to be persistent and/or consistent (decision diamond 220), one or more of steps 224, 228, 232, and 236 is performed.

In step 224, the accessibility expert 106 provides an instructional message to the selected user. As noted, the instructional message can educate the selected user on his or her rights and accommodations offered by the enterprise to address any impairment associated with the disability.

In step 228, the accessibility access expert 108 recommends a feature, setting, and/or configuration of a device associated with the selected user. By way of example, the accessibility access expert 108 can alert the selected user to the availability of mandated (e.g., by Section 508 of the Rehabilitation Act Amendments of 1998, Section 255 of the Telecommunication Act of 1996, and the Communications and Video Accessibility Act of 2010) support for the user's disability. In telecommunication equipment and services, support for TTY communication by deaf users must be provided. Support for hard-of-hearing users, in the form of user-adjustable increased amplification, must be provided. Support for blind users, such as the availability of spoken caller ID and a control mechanism with tactilely discernible keys, must be provided. Support for low-vision users, in the form of large-font displays, must be provided.

In step 232, the data collection and analysis module 116 updates the enterprise and/or compliance secure databases to reflect the collected, processed, and analyzed data regarding the selected user.

In step 236, the reporting module 112 generates a report mapping the previously known and newly discovered qualified disabilities to job classifications and preserves the record(s) for mandatory reporting, such as to a governmental entity. The format of the report can vary by the requirements of the government entity associated with the server 152 and to comply with confidentiality requirements.

### Automatic Device Reconfiguration

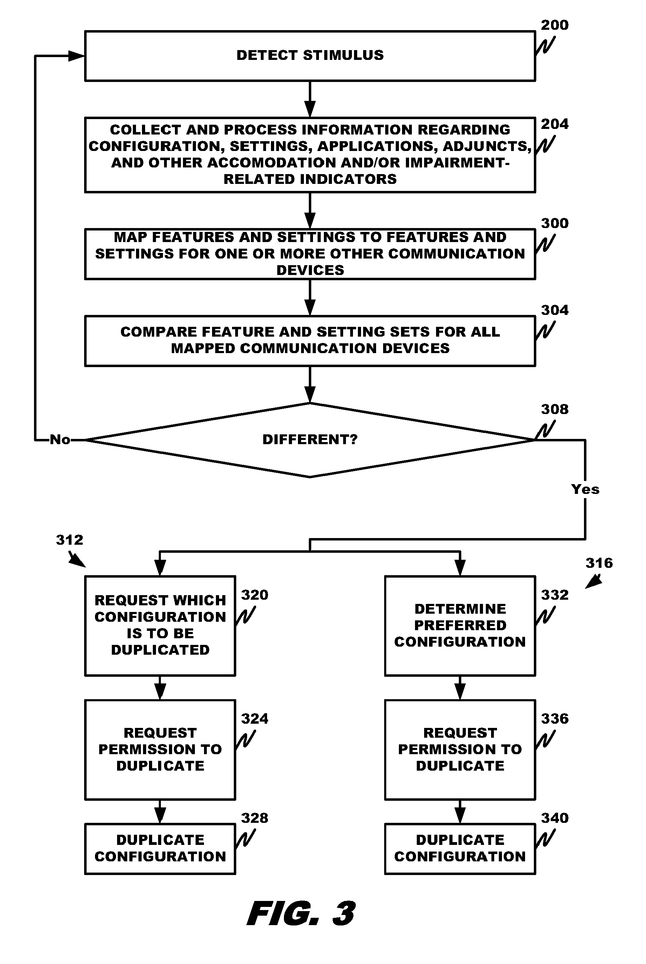

With reference to Fig. 3, an operation of the accessibility access expert 108.

Steps 200 and 204 were described above with reference to Fig. 2.

In step 300, the accessibility access expert 108 maps features and settings to similar features and settings for one or more other communication devices. Because the codes and/or values for features and settings can change from device-to-device and from manufacturer-to-manufacturer and involve differing syntax and format, conversion tables are typically employed to map a first feature, such as screen magnification, in a first device to the first feature in a different second device and to map a first setting value, such as volume level or font size, in the first device to a similar first setting value in the second device. Such mapping can be performed using the techniques set forth in US 8,219,512, 8,027,946, and 7,873,992, each of which is incorporated herein by this reference.

In step 304, the accessibility access expert 108, using the mapped information (which defines features and settings in a common language or using a common syntax and format) compares feature and setting sets for all mapped communication devices and, in decision diamond 308, determines, with respect to selected features and values, whether or not the devices have differing enabled or disabled features and feature values.

When there are no differences for the selected features and settings (meaning that the selected feature is enabled or disabled in each of the devices and the settings have substantially the same values), the accessibility access expert 108 returns to step 200.

When there is at least one difference for the selected features and settings (meaning that the selected feature is enabled or disabled in one device and disabled or enabled in another device and/or that one or more settings have substantially different values in different devices), the accessibility access expert 108 can perform either task set 312 or 316. The expert 108 can further provide the user with a message, such as discussed above, inviting self-identification of a potential impairment or disability requiring accommodation.

With reference to task set 312, the accessibility access expert 108, in step 320, requests the user, via a user interface, whether duplication is desired and which configuration is to be duplicated. In step 324, when the user indicates that duplication is desired and which configuration is to be duplicated to one or more other devices, the expert 108 requests permission, via the user interface, to initiate duplication. When permission is received from the user, the expert 108, in step 328, duplicates the configuration in the other or target device. This typically requires the common or universal language to be converted into the language used by that device to define its features and settings.

With reference to task set 316, the accessibility access expert 108, in step 332, determines, based on user behavior, which of device configurations (or feature and setting sets) in use by the various devices of the user is preferred. This determination typically assumes that the device most commonly used by the user, such as a smart phone, has the preferred configuration. In step 336, the expert requests the user, via the user interface, permission to duplicate the preferred configuration to a target device. When the user indicates that duplication is desired, the expert 108, in step 340, duplicates the configuration in the target device.

### Disability Monitoring of Third Parties

Existing accommodations of impairments and/or disabilities can be identified for third parties (or non-enterprise parties) using features, settings, and assistive technologies determined using publicly available information. Such information is typically available via a web browser 156 and 176 used by the third party user. The settings and/or enabled or disabled features of the web browser or type of web browser used can indicate a user accommodation and/or impairment and disability. By way of example, a server can distinguish between a web browser user using a mouse as opposed to a web browser user using a keyboard to navigate a web page. A person without a disability is likely to use a mouse because of speed and convenience; whereas someone will use a keyboard because he or she cannot work a mouse. In other examples, a server can detect one or more of mouse keys, web browser requested web page font selection and formatting, web browser requested web page font, text, and/or icon size, web browser requested text and/or background colors for the web page, web browser requested contrast for the web page, web browser request to disablement of unnecessary web page animations, web browser requested removal of background images in the web page, use of keyboard shortcuts by the web browser user, and web browser use of Internet accessibility.

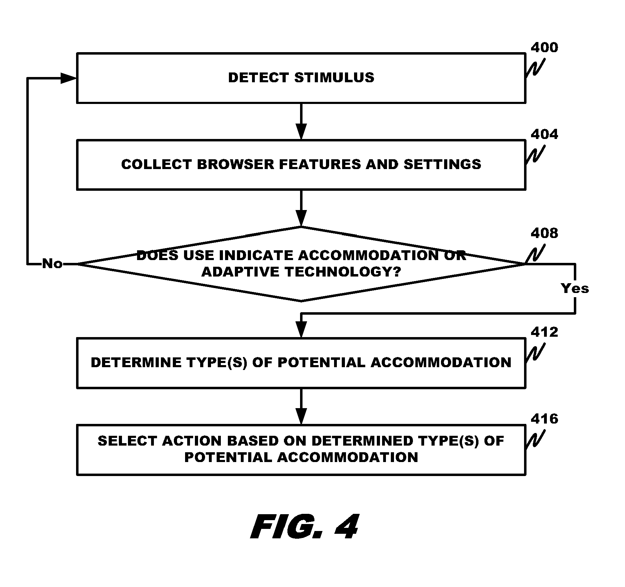

With reference to Fig. 4, the data collection and analysis module 116 detects a stimulus in step 400, such as a web browser requesting a web page or inputting information into a web page.

In step 404, the data collection and analysis module 116 collects and processes browser enabled or disabled features and settings.

In decision diamond 408, the collection and analysis module 116 determines, based on the collected and processed features and settings, whether or not the web browser user is utilizing an accommodation generally associated with an impairment and/or disability. If not, the module 116 returns to step 400. If so, the module 116, in step 412, determines a potential type of accommodation of the user and/or a possible or probable type of impairment and/or disability of the user.

In step 416, the accessibility expert 106 selects an action to be performed based on the determined type(s) of potential accommodation and/or disability. Actions include, in a contact center, providing the user with a human or automated agent specially skilled to interact with a user having the potential impairment and/or disability, in a job application setting, encouraging the user to indicate in a job application that he or she has the impairment and/or disability, and in a commercial web site, providing the user with targeted advertisements regarding products or services for users having the impairment and/or disability. For example, targeted advertising can be conducted by services such as Google based not only on search history or social media interactions but also on the automated detection of user-specified accessibility preferences and other web browser features and settings to target individuals with a specific type of impairment or disability. Other actions are possible depending on the context and/or purpose of the interaction between the web browser and server.

The exemplary systems and methods of this disclosure have been described in relation to a distributed processing network. However, to avoid unnecessarily obscuring the present disclosure, the preceding description omits a number of known structures and devices. This omission is not to be construed as a limitation of the scopes of the claims. Specific details are set forth to provide an understanding of the present disclosure. It should however be appreciated that the present disclosure may be practiced in a variety of ways beyond the specific detail set forth herein.

Furthermore, while the exemplary aspects, embodiments, and/or configurations illustrated herein show the various components of the system collocated, certain components of the system can be located remotely, at distant portions of a distributed network, such as a LAN and/or the Internet, or within a dedicated system. Thus, it should be appreciated, that the components of the system can be combined in to one or more devices, such as a server, or collocated on a particular node of a distributed network, such as an analog and/or digital telecommunications network, a packet-switch network, or a circuit-switched network. It will be appreciated from the preceding description, and for reasons of computational efficiency, that the components of the system can be arranged at any location within a distributed network of components without affecting the operation of the system. For example, the various components can be located in a switch such as a PBX and media server, gateway, in one or more communications devices, at one or more users' premises, or some combination thereof. Similarly, one or more functional portions of the system could be distributed between a telecommunications device(s) and an associated computing device.

Furthermore, it should be appreciated that the various links connecting the elements can be wired or wireless links, or any combination thereof, or any other known or later developed element(s) that is capable of supplying and/or communicating data to and from the connected elements. These wired or wireless links can also be secure links and may be capable of communicating encrypted information. Transmission media used as links, for example, can be any suitable carrier for electrical signals, including coaxial cables, copper wire and fiber optics, and may take the form of acoustic or light waves, such as those generated during radio-wave and infra-red data communications.

Also, while the flowcharts have been discussed and illustrated in relation to a particular sequence of events, it should be appreciated that changes, additions, and omissions to this sequence can occur without materially affecting the operation of the disclosed embodiments, configuration, and aspects.

A number of variations and modifications of the disclosure can be used. It would be possible to provide for some features of the disclosure without providing others.

For example in one alternative embodiment, the systems and methods of this disclosure can be implemented in conjunction with a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device or gate array such as PLD, PLA, FPGA, PAL, special purpose computer, any comparable means, or the like. In general, any device(s) or means capable of implementing the methodology illustrated herein can be used to implement the various aspects of this disclosure. Exemplary hardware that can be used for the disclosed embodiments, configurations and aspects includes computers, handheld devices, telephones (e.g., cellular, Internet enabled, digital, analog, hybrids, and others), and other hardware known in the art. Some of these devices include processors (e.g., a single or multiple microprocessors), memory, nonvolatile storage, input devices, and output devices. Furthermore, alternative software implementations including, but not limited to, distributed processing or component/object distributed processing, parallel processing, or virtual machine processing can also be constructed to implement the methods described herein.

In yet another embodiment, the disclosed methods may be readily implemented in conjunction with software using object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosure is dependent on the speed and/or efficiency requirements of the system, the particular function, and the particular software or hardware systems or microprocessor or microcomputer systems being utilized.

In yet another embodiment, the disclosed methods may be partially implemented in software that can be stored on a storage medium, executed on programmed general-purpose computer with the cooperation of a controller and memory, a special purpose computer, a microprocessor, or the like. In these instances, the systems and methods of this disclosure can be implemented as program embedded on personal computer such as an applet, JAVA® or CGI script, as a resource residing on a server or computer workstation, as a routine embedded in a dedicated measurement system, system component, or the like. The system can also be implemented by physically incorporating the system and/or method into a software and/or hardware system.

Although the present disclosure describes components and functions implemented in the aspects, embodiments, and/or configurations with reference to particular standards and protocols, the aspects, embodiments, and/or configurations are not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various aspects, embodiments, and/or configurations, includes components, methods, processes, systems and/or apparatus substantially as depicted and described herein, including various aspects, embodiments, configurations embodiments, subcombinations, and/or subsets thereof. Those of skill in the art will understand how to make and use the disclosed aspects, embodiments, and/or configurations after understanding the present disclosure. The present disclosure, in various aspects, embodiments, and/or configurations, includes providing devices and processes in the absence of items not depicted and/or described herein or in various aspects, embodiments, and/or configurations hereof, including in the absence of such items as may have been used in previous devices or processes, e.g., for improving performance, achieving ease and\or reducing cost of implementation.

The foregoing discussion has been presented for purposes of illustration and description. The foregoing is not intended to limit the disclosure to the form or forms disclosed herein. In the foregoing Detailed Description for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A method, comprising:
collecting, by a microprocessor executable data collection and analysis module, accommodation and/or impairment-related information associated with one or more computational devices associated with a user;
determining, by a microprocessor executable data collection and analysis module and based on the collected accommodation and/or impairment-related information, that the user potentially has an impairment and/or disability requiring accommodation; and
performing an action associated with the determination that the user potentially has an impairment and/or disability requiring accommodation and/or is using an accommodation for an impairment and/or disability.

2. The method of claim 1, wherein the one or more computational devices comprise a telecommunication device, wherein the accommodation and/or impairment-related information comprises one or more of an enabled feature, a disabled feature, a setting, an assistive technology and a pattern of behavior of the user, wherein the action comprises one or more of provide an instructional message to the selected user, recommend a feature, setting, and/or configuration of a computational device associated with the user, and track the accommodation and/or impairment and/or disability for reporting to a governmental entity upon request, wherein the accommodation and/or impairment-related information relates to use by the user of one or more of sticky keys, toggle keys, filter keys, mouse keys, sound or volume settings, font selection, formatting, font, text, and/or icon size, screen magnification, visual indication of sounds, changed computer sounds, text and/or background colors, contrast, color and transparency of window borders, thickness of focus rectangle, disablement of unnecessary animations, read mode, speak, remove background images, narration, audio description, on-screen keyboard, color, size, and/or thickness of the on-screen mouse pointer, scroll speed of the mouse wheel, keyboard settings, keyboard shortcuts, time period for how long notification dialog boxes remain open, Internet accessibility, a screen magnifier, a screen reader, a Braille printer, a Braille note taker, a Braille embosser, a book reader, an add-in to convert documents to DAISY format for a book reader, a speech synthesizer, a speech recognition program, a touch keyboard, an on-screen keyboard, a mouse, a joystick, a trackball, a keyboard filter, an alternative PC hardware or all-access workstations, an alternative input device, a sign language interpretation device, a personal listening device, a personal amplification device, a sign language translator, a word prediction program, a reading tool, a learning disability program, an augmentative and assistive communication device, a bone-conduction phone, an amplified phone, Universal Access Phone Status™, a text telephone, a pattern of behavior of the user, and a TTY user interface for a voice messaging system, wherein the determining step determines whether a pattern of behavior of the user comprises multiple different types of accommodation and/or impairment-related information, and wherein the determining step comprises at least one of the sub steps of determining a type of the accommodation and/or impairment and/or disability, determining a severity of the accommodation and/or impairment and/or disability, and determining a likelihood that the user has the impairment and/or disability requiring accommodation (and therefore, tracking).

3. The method of claim 1 or claim 2, wherein the determining step comprises at least one of the sub steps of determining whether the collected accommodation and/or impairment-related information is attributable to the user or a physical environment of the user and determining whether the collected accommodation and/or impairment-related information has occurred for at least a predetermined period of time, wherein the action is one or more of providing the user with a human or automated agent specially skilled to interact with the user, encouraging the user to indicate in a job application that he or she has the accommodation and/or impairment and/or disability, and providing the user with targeted advertisements regarding products or services for users having the impairment and/or disability, and further comprising:
mapping enabled and disabled features and/or settings of a first communication device associated with the user to the features and/or settings of a second communication device associated with the user; and
changing at least some of the enabled and disabled features and/or settings of the second communication device to corresponding ones of enabled and disabled features and/or settings of the first communication device.

4. A non-transient and tangible computer readable medium, comprising microprocessor executable instructions that, when executed by the microprocessor perform steps, including:
collect accommodation and/or impairment-related information associated with one or more computational devices associated with a user;
determine that the user potentially has an accommodation and/or impairment and/or disability requiring accommodation; and
perform an action associated with the determination that the user potentially has an impairment and/or disability requiring accommodation and/or is using an accommodation for an impairment and/or disability.

5. The computer readable medium of claim 4, wherein the accommodation and/or impairment-related information comprises one or more of an enabled feature, a disabled feature, a setting, an assistive technology, and a pattern of behavior of the user, wherein the action comprises one or more of provide an instructional message to the selected user, recommend a feature, setting, and/or configuration of a computational device associated with the user, and track the accommodation and/or impairment and/or disability for reporting to a governmental entity upon request, and wherein the accommodation and/or impairment-related information relates to use by the user of one or more of sticky keys, toggle keys, filter keys, mouse keys, sound or volume settings, font selection, formatting, font, text, and/or icon size, screen magnification, visual indication of sounds, changed computer sounds, text and/or background colors, contrast, color and transparency of window borders, thickness of focus rectangle, disablement of unnecessary animations, read mode, speak, remove background images, narration, audio description, on-screen keyboard, color, size, and/or thickness of the on-screen mouse pointer, scroll speed of the mouse wheel, keyboard settings, keyboard shortcuts, time period for how long notification dialog boxes remain open, Internet accessibility, a screen magnifier, a screen reader, a Braille printer, a Braille note taker, a Braille embosser, a book reader, an add-in to convert documents to DAISY format for a book reader, a speech synthesizer, a speech recognition program, a touch keyboard, an on-screen keyboard, a mouse, a joystick, a trackball, a keyboard filter, an alternative PC hardware or all-access workstations, an alternative input device, a sign language interpretation device, a personal listening device, a personal amplification device, a sign language translator, a word prediction program, a reading tool, a learning disability program, an augmentative and assistive communication device, a bone-conduction phone, an amplified phone, Universal Access Phone Status™, a text telephone, and a TTY user interface for a voice messaging system.

6. The computer readable medium of claim 4 or claim 5, wherein at least one of the following is true: (a) the determining step comprises at least one of the sub steps of determining whether the collected accommodation and/or impairment-related information is attributable to the user or a physical environment of the user and determining whether the collected accommodation and/or impairment-related information has occurred for at least a predetermined period of time and (b) the determining step comprises at least one of the sub steps of determining a type of the accommodation and/or impairment and/or disability, determining a severity of the impairment and/or disability, and determining a likelihood that the user has the impairment and/or disability.

7. The computer readable medium of any one of claims 4 to 6, wherein the one or more computational devices comprise a telecommunication device and further comprising the steps:
mapping enabled and disabled features and/or settings of a first communication device associated with the user to the features and/or settings of a second communication device associated with the user; and
changing at least some of the enabled and disabled features and/or settings of the second communication device to corresponding ones of enabled and disabled features and/or settings of the first communication device, wherein the action is one or more of providing the user with a human or automated agent specially skilled to interact with the user, encouraging the user to indicate in a job application that he or she has the accommodation and/or impairment and/or disability, and providing the user with targeted advertisements regarding products or services for users having the impairment and/or disability.

8. A system, comprising:
a microprocessor executable data collection and analysis module operable to collect accommodation and/or impairment-related information associated with one or more computational devices associated with a user and to determine, based on the collected accommodation and/or impairment-related information, that the user potentially has an impairment and/or disability requiring accommodation; and
at least one other module to perform an action associated with the determination that the user potentially has an impairment and/or disability requiring accommodation and/or is using an accommodation for an impairment and/or disability.

9. The system of claim 8, wherein the accommodation and/or impairment-related information comprises one or more of an enabled feature, a disabled feature, a setting, an assistive technology, and a pattern of behavior of the user, wherein the at least one other module comprises at least one of:
an accessibility expert operable to provide an instructional message to the selected user;
an accessibility access expert operable to recommend a feature, setting, and/or configuration of a computational device associated with the user; and
a reporting module operable to report the impairment and/or disability to a governmental entity, wherein the accommodation and/or impairment-related information relates to use by the user of one or more of sticky keys, toggle keys, filter keys, mouse keys, sound or volume settings, font selection, formatting, font, text, and/or icon size, screen magnification, visual indication of sounds, changed computer sounds, text and/or background colors, contrast, color and transparency of window borders, thickness of focus rectangle, disablement of unnecessary animations, read mode, speak, remove background images, narration, audio description, on-screen keyboard, color, size, and/or thickness of the on-screen mouse pointer, scroll speed of the mouse wheel, keyboard settings, keyboard shortcuts, time period for how long notification dialog boxes remain open, Internet accessibility, a screen magnifier, a screen reader, a Braille printer, a Braille note taker, a Braille embosser, a book reader, an add-in to convert documents to DAISY format for a book reader, a speech synthesizer, a speech recognition program, a touch keyboard, an on-screen keyboard, a mouse, a joystick, a trackball, a keyboard filter, an alternative PC hardware or all-access workstations, an alternative input device, a sign language interpretation device, a personal listening device, a personal amplification device, a sign language translator, a word prediction program, a reading tool, a learning disability program, an augmentative and assistive communication device, a bone-conduction phone, an amplified phone, Universal Access Phone Status™, a text telephone, a pattern of behavior of the user, and a TTY user interface for a voice messaging system, wherein the one or more computational devices comprise a telecommunication device, wherein the determining step determines whether a pattern of behavior of the user comprises multiple different types of accommodation and/or impairment-related information, and wherein the determining operation comprises at least one of the sub-operations of determining whether the collected accommodation and/or impairment-related information is attributable to the user or a physical environment of the user and determining whether the collected accommodation and/or impairment-related information has occurred for at least a predetermined period of time.

10. The system of claim 8 or claim 9, wherein the determining operation comprises at least one of the sub-operations of determining a type of the accommodation and/or impairment and/or disability, determining a severity of the impairment and/or disability, and determining a likelihood that the user has the impairment and/or disability, wherein the at least one other module is an accessibility access expert operable to:
map enabled and disabled features and/or settings of a first communication device associated with the user to the features and/or settings of a second communication device associated with the user; and
change at least some of the enabled and disabled features and/or settings of the second communication device to corresponding ones of enabled and disabled features and/or settings of the first communication device, and wherein the action is one or more of providing the user with a human or automated agent specially skilled to interact with the user, encouraging the user to indicate in a job application that he or she has the accommodation and/or impairment and/or disability, and providing the user with targeted advertisements regarding products or services for users having the impairment and/or disability.
